# EUROPEAN PATENT SPECIFICATION

(11) **EP 2 923 694 B1**
(45) Date of publication and mention of the grant of the patent: **14.11.2018**
(21) Application number: 15160976.5
(22) Date of filing: 26.03.2015
(51) Int. Cl.: A61K 9/08, A61K 9/00, A61K 47/18, A61K 31/195

(54) **ORAL LIQUID PHARMACEUTICAL SOLUTION OF GABAPENTIN**
ORALE FLÜSSIGE PHARMAZEUTISCHE LÖSUNG VON GABAPENTIN
SOLUTION PHARMACEUTIQUE LIQUIDE ORALE DE GABAPENTINE

(30) Priority: 27.03.2014 TR 201403582
(43) Date of publication of application: 30.09.2015
(73) Proprietor: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Tombayoglu, Vildan, 34460 Istanbul (TR); Sezgin, Asiye, 34460 Istanbul (TR); Gökcek, Sevgi, 34460 Istanbul (TR)
(74) Representative: Sevinç, Erkan

(56) References cited:
- EP-B1- 1 077 692
- WO-A1-02/094220
- WO-A2-2007/107835
- US-A- 4 960 931

## Description

### Field of Invention

The present invention relates to an oral liquid pharmaceutical solution comprising gabapentin or a pharmaceutically acceptable salt, solvate or hydrate thereof and disodium EDTA and at least one pharmaceutically acceptable excipient.

### Background of Invention

Gamma-Aminobutyric acid (GABA) is an inhibitory amino acid found in the mammalian central nervous system (CNS). It has been reported that dysfunctions with GABA neurotransmission in the CNS may contribute or even cause psychiatric and neurological diseases such as epilepsy, schizophrenia, Parkinson's disease, Huntington's Chorea and dyskinesia (Saletu B., et al., International Journal of Clinical Pharmacology, Therapy, and Toxicology, 1986;24:362-373).

Gabapentin (I-(aminomethyl)-cyclohexaneocetic acid)'s chemical structure is illustrated with Formula I given below.

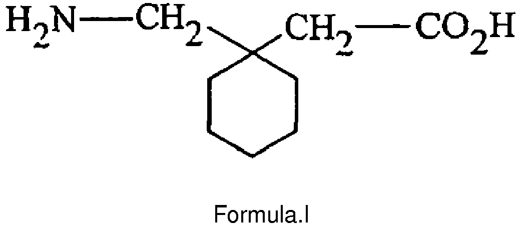

Gabapentin was designed as a GABA analog that would cross the blood-brain barrier. Gabapentin was found to have anticonvulsant and antispastic activity with extremely low toxicity in human. Gabapentin is presently marketed under the trademark Neurontin as adjunctive therapy in the treatment of partial seizures in patients with epilepsy.

In prior art, US Patent Numbers 4, 024, 175 and 4, 087, 544 disclose the use of gabapentin for the treatment of certain forms of epilepsy, faintness attacks, hypokinesia, and cranial traumas. Additionally, gabapentin brings about an improvement of cerebral functions and thus is useful in treating geriatric patients. US Patent Number 5, 084, 479 discloses the use of gabapentin in neurodegenerative disorders. US Patent Number 5, 025, 035 discloses the use of gabapentin in treating depression; US Patent Number 5, 510, 381 discloses the use of gabapentin in treating mania and bipolar disorders. US Patent Number 5, 792, 796 discloses the use of gabapentin in treating anxiety and panic. US Patent Number 6, 127, 418 discloses the use of gabapentin in treating gastrointestinal damage; US Patent Numbers 4, 894, 476 and 4, 960, 931 disclose a novel crystalline monohydrate form of gabapentin; and US Patent Numbers 5, 133, 451; 5,319,135; 5,362,883; 5,693,845; 5, 091, 567 and 5, 068, 413 disclose processes for preparing gabapentin as well as intermediates used in these processes.

WO 2007/107835 A2 discloses liquid formulations comprising antiepileptic drugs; in particularly GABA analogues. The use of a polyhydric alcohol containing 2 to 6 carbon atoms is suggested in order to solve stability related problems.

Solid oral dosage forms have the largest and most important role in the entire pharmaceutical formulations. However, there are a huge number of patients who have difficulties swallowing these dosage forms is not less. Especially, for psychiatric patients who reject to take medicine, therapeutic agents can be administrated in different forms such as solutions, suspensions, elixirs and emulsions. Patients who have difficulties in swallowing the solid forms are in need of oral solution forms of gabapentin.

In addition to this, oral solutions provide flexibility in dosage regimes for patients who need special doses of the drug. Oral liquid dosage forms are chosen by patients who are travelling or have little access to water or patients who are mentally retarded or nauseated. Unlike the solid dosage forms, oral liquid pharmaceutical forms require no dissolution so its absorption is rapid and unaffected by alterations in gastric pH. Thus, they deliver high concentration of active pharmaceutical agent to the intestinal epithelium and minimize first-pass metabolism.

On the other side, there are also a number of "challenges" surrounding the formulation and development of these forms such as stability, solubility and taste. Moreover, active pharmaceutical agents in liquid forms are more susceptible to chemical and physical instability than the solid state. In addition to the solubility, gabapentin needs to be physically and chemically stable in the oral solution. Trace amounts of impurities from active pharmaceutical agents or excipients and the pH of the solution may cause the degradation of active pharmaceutical agent and this may cause an increase in instability when the solution is consistently introduced into atmosphere.

In this invention, to fulfill requirements of the oral liquid pharmaceutical solution of gabapentin, a formulation have been developed comprising gabapentin or a pharmaceutically acceptable salt, solvate or hydrate thereof and disodium EDTA and at least one or more pharmaceutically acceptable excipients. To further ensure the stability and better taste of formulation, disodium EDTA has been used as antioxidant.

### Description of Invention

The term "gabapentin" as used herein refers to gabapentin as well as pharmaceutically acceptable forms as crystalline, hydrate or anhydrous polymorph forms.

The main object of the present invention is to obtain an oral pharmaceutical formulation of gabapentin in solution form wherein gabapentin and excipients are dissolved homogeneously.

Another object of the present invention is to obtain an oral pharmaceutical solution providing rapid and high absorption of gabapentin which is already dissolved in the solution. Another object of the present invention is to provide a stable oral pharmaceutical solution of gabapentin.

There is also provided an oral pharmaceutical solution of gabapentin which can be administered orally to patients who have difficulty in swallowing.

There is also provided an oral pharmaceutical solution of gabapentin wherein flexible dosing is possible.

There is also provided an oral taste masked pharmaceutical solution of gabapentin.

There is also provided a homogeneous and stable pharmaceutical solution of gabapentin suitable for oral administration to a mammal.

In this invention, the oral liquid pharmaceutical solution comprises gabapentin and disodium EDTA and at least one pharmaceutically acceptable excipient. The chemical stability of gabapentin has been provided throughout the shelf life by disodium EDTA; precipitation and crystallization in the solution has been prevented while unpleasant taste problem has been overcome.

It has been surprisingly found that a stable and homogenous solution of gabapentin can be prepared with disodium EDTA as an antioxidant to enhance physical and chemical stability of gabapentin in the present oral solution. The pharmaceutical formulation disclosed herein is a stable solution wherein gabapentin or pharmaceutically acceptable excipients are not precipitated.

The oral liquid pharmaceutical solution of this invention comprises one or more pharmaceutically acceptable excipient which is selected from the group comprising co-solvents, solvents, antioxidants, microbial preservatives, buffering agents, aromatic agents, sweeteners and diluents.

Co-solvents and solvents may include but not limited to glycerine, alcohols, propylene glycol, polyethylene glycol, benzyl alcohol ,water, ethanol, isopropyl alcohol or their mixtures thereof. The development of the gabapentin oral solution of the present invention, it was shown that theoretically most alcohols possess a preserving and stabilizing action in aqueous solution. However, certain alcohols had undesirable properties and were not useful in the present pharmaceutical compositions. For example, ethyl alcohol is not desirable for pediatric formulations, propylene glycol and benzyl alcohol have an unpleasant taste.

Suitable antioxidants may include but not limited to butylated hydroxyanisole (BHA), butylated hydroxytoluene (BHT), ascorbic acid, beta-carotene, alpha-tocopherol, propyl gallate, gentisic acid sodium ascorbate, sodium bisulfite, sodium metabisulfite, monothioglycero, cysteine, thioglycolate sodium, acetone sodium bisulfite, ascorbate (sodium/acid), bisulfite sodium, cystein/cysteinate HCI, dithionite sodium (Na hydrosulfite, Na sulfoxylate), gentisic acid, gentisic acid ethanolamine, glutamate monosodium, formaldehyde sulfoxylate sodium, metabisulfite potassium, metabisulfite sodium, monothioglycerol (thioglycerol), propyl gallate, sulfite sodium, tocopherol alpha, thioglycolate sodium, EDTA in calcium and sodium compounds_or the mixtures thereof.

In this invention, disodium EDTA is used as an antioxidant in the oral liquid pharmaceutical solution. EDTA (Ethylenediaminetetraacetic acid) is a chemical that binds and holds on to (chelates) minerals and metals such as chromium, iron, lead, mercury, copper, aluminum, nickel, zinc, calcium, cobalt, manganese, and magnesium. When they are bound, they can't have any effects on the body and they are removed from the body. EDTA is used in calcium and sodium compounds to improve stability in pharmaceutical products.

Microbial preservatives may include but not limited to sodium benzoate, benzoic acid, boric acid, sorbic acid and their salts thereof, benzyl alcohol, benzalkonium chloride, parahydroxybenzoic acids and their alkyl esters, methyl and propyl parabens or their mixtures thereof.

In this invention, sodium benzoate is preferably used as a microbial preservative in the oral liquid pharmaceutical solution. Sodium benzoate is the sodium salt of benzoic acid. It is an antimicrobial preservative and flavoring agent. It is bacteriostatic and fungistatic under acidic conditions. Concentration as a preservative is limited by the FDA in the U.S. to 0.1% by weight. Sodium benzoate is a well known and widely used preservative for pharmaceutical products such as syrups, flavored vehicles, and multiple dose containers for liquid preparations. The pH is an important factor and use levels may vary. The advantages of sodium benzoate are that it is colorless, odorless, readily soluble, and generally is compatible with other ingredients.

Buffering agents may include but not limited to ascorbic acid, acetic acid, tartaric acid, citric acid monohydrate, trisodium citrate dehydrate, sodium citrate, potassium citrate, sodium phosphate, tricalcium phosphate, calcium carbonate, sodium bicarbonate, calcium phosphate, carbonated calcium phosphate, magnesium hydroxide, hydrochloric acid, sodium hydroxide or their mixtures thereof.

Diluents may include but not limited to maltitol solution, glucose syrup, glycerin, sorbitol and mannitol solutions, sucrose, sorbitol, xylitol, dextrose, fructose, sugar potassium, aspartame, saccharine, saccharine sodium, spray dried or anhydrase lactose, mannitol, starch or their mixtures thereof.

In this invention, maltitol solution is preferably used as a diluent in the oral liquid pharmaceutical solution. We have surprisingly and unexpectedly found that maltitol doesn't only act as a diluent but it also has preservative and a stabilizing effect on gabapentin and it substantially masks the bitter taste of the gabapentin as a result of its sweet taste and additionally is non-cariogenic. Maltitol is used as a low-calorie sweetening agent, it's slowly absorbed and lesser effect on blood glucose which makes it somewhat more suitable for people. It is not metabolized by oral bacteria, so it does not promote tooth decay. It has an advantage that crystallization is less likely. When maltitol solution more than %75 by weight was added, the bitter taste of gabapentin was masked the present composition had an agreeable taste and unpleasant taste problems have been achieved with less sweeteners.

Sweeteners may include but not limited to sucralose, aspartame, acesulfame-K, thaumatin, mogroside, saccharin and salts thereof, sodium cyclamate, glucose, sucrose, lactose, fructose, mannitol, sorbitol, lactitol, xylitol, erythritol, glycyrrhizin, monosodium glycyrrhizinate, monoamonium glycyrrhizinate, isomalt, glycerine, dextrose or their mixtures thereof.

In this invention, sucralose is preferably used as a sweetener in the oral liquid pharmaceutical solution.

Aromatic agents may include but not limited to fruit aromas such as orange, banana, strawberry, cherry, wild cherry, lemon and the like, and other aromas such as cardamom, anis, mint, menthol, vanillin or their mixtures thereof.

In this invention, orange aroma is preferably used as an aromatic agent in the oral liquid pharmaceutical solution.

In this invention, the oral liquid pharmaceutical solution comprises gabapentin, disodium EDTA, sodium benzoate and at least one pharmaceutically acceptable excipient. Antioxidants are used in pharmaceutical solutions to enhance the stability of active agents and excipients that are susceptible to chemical degradation by oxidation. The oxidation of gabapentin in an oral liquid pharmaceutical solution is difficult to control due to its chemical properties. In this present invention, to prevent oxidative degradation of gabapentin and further ensure the stability, disodium EDTA was used. Disodium EDTA inhibits free radical-induced drug decomposition and prevent spoilage by reacting with oxygen and it slows the development of off-flavours, odours and colour changes caused by oxidation. Sodium benzoate has also bacteriostatic and fungistatic activities against bacterial and fungal contamination so it enhances the stability of the gabapentin oral solution formulation.

According to the embodiments of this invention mentioned above, disodium EDTA and sodium benzoate provides a synergistic effect and a stable oral liquid pharmaceutical solution comprising gabapentin without any precipitation, degradation, unpleasant taste or microbial problems have been achieved.

According to an embodiment of the present invention, said oral pharmaceutical solution contains gabapentin in an amount of 1% to 10% of total weight of the pharmaceutical solution, more preferably 4% to 6% of total weight of the pharmaceutical solution.

As another embodiment of the invention, disodium EDTA is present in the range of 0.05% to 0.1% of total weight of the pharmaceutical solution, more preferably 0.09% to 0.1% of total weight of the pharmaceutical solution.

As another embodiment of the invention, sodium benzoate is present in the range of 0,01% to 0.1% of total weight of the pharmaceutical solution, more preferably 0.01% to 0.03% of total weight of the pharmaceutical solution

As a further embodiment of the invention, the weight ratio of disodium EDTA to Gabapentin is preferably 1:50 and sodium benzoate to disodium EDTA is preferably 1:5

According to an embodiment of the present invention, said oral liquid pharmaceutical solution of gabapentin comprises;
a) 1 to 10% by weight of gabapentin,
b) 0.01 to 0.1% by weight of sodium benzoate,
c) 10 to 25% by weight of maltitol solution,
d) 0.05 to 0.1% by weight of disodium EDTA,
e) 0.05 to 0.5% by weight of sucralose,
f) 0.1 to 1 % by weight of orange aroma.

### Example: Gabapentin oral solution

| **Ingredients** | **Amount %** |
|---|---|
| Gabapentin | 4 - 6 |
| Sodium benzoate | 0.01 - 0.03 |
| Maltitol solution | 18 - 20 |
| Disodium EDTA | 0.09 - 0.1 |
| Sucralose | 0.1 - 0.3 |
| Orange aroma | 0.7 - 0.9 |
| Distilled water | q.s. |

| | |
|---|---|
| q.s.: quantum sufficient | |

The process of the formulations is carried out under nitrogen gas as follows: Maltitol solution is added in a tank containing water. Sodium benzoate and disodium EDTA are added to this solution and mixed until homogenous mixture is observed. Gabapentin is added to this solution and mixed. All other excipients are added to this mixture and mixed until complete dissolution is achieved. The resulting mixture filtrated and the mixture is filled into bottles.

Stability tests results of the Gabapentin oral solution at two different conditions example are provided below. After storage at (25 ± 2) °C / % (60 ± 5)RH for 6 months and at (5 ± 3) °C for 6 monts, test results demonstrate that the disclosed oral solution of Gabapentin is stable during the shelf life.

### Stability test conditions and periods

| **Storage condition: Temperature (°C)** | **Storage condition: Relative Humidity (%)** | **Duration (Months)** |
|---|---|---|
| 5°C ± 3°C | - | 0. 3, 6 |
| 25°C ± 2°C | % 60 ± %5 | 0, 3, 6 |

### Stability test results at 5°C ± 3°C

| **TESTS** | **SPECIFICATIONS** | **Origin** | **3 Month 5°C** | **6 Month 5°C** |
|---|---|---|---|---|
| **Impurity** | Laktam impurity; max. 0.40% For each unknown impurities, max 0.20% Total impurity; max. 1.0% | 0.035% | 0.04% | 0.05% |
| | | ND* | 0.02% | 0.06% |
| | | 0.035% | 0.06% | 0.13% |
| **Quantity of Gabapentin** | 250.0 mg Gabapentin / 5 mL ± 10.0% (225.0 mg/ 5 mL - 275.0 mg/ 5 mL) | 252.5 mg/5 mL | 251.9 mg/5 mL | 249.8 mg/5 mL |

| | | | | |
|---|---|---|---|---|
| *ND: Not Detected | | | | |

### Stability test results 25°C ± 2°C

| **TESTS** | **SPECIFICATIONS** | **Origin** | **3 Month** 25°C 60% RH | **6 Month** 25°C 60% RH |
|---|---|---|---|---|
| **Impurity** | Laktam impurity; max. 0.40% For each unknown impurities; max. 0.20% Total impurity; max. 1.0% | 0.035% | 0.10% | 0.30% |
| | | ND* | 0.04% | 0.16% |
| | | 0.0035% | 0.17% | 0.72% |
| **Quantity of Gabapentin** | 250.0 mg Gabapentin / 5 mL ± 10.0% (225.0 mg/ 5 mL - 275.0 mg/ 5 mL) | 252.5 mg/5 mL | 249.9 mg/5 mL | 246.2 mg/5 mL |

| | | | | |
|---|---|---|---|---|
| *ND: Not Detected | | | | |

### Stability tests methods

Quantity of gabapentin and impurity tests were determined by HPLC method in which column: Synergi Fusion 80A 250x4.6 mm x 4.0 µm or Atlantis dC18, 5 µm, 4.6 mm x 250 mm ; injection volume: 10 microliters ; wavelength: UV, 210 nm ; column temperature: 45°C.

## Claims

1. An oral liquid pharmaceutical solution comprising gabapentin and disodium EDTA and at least one pharmaceutically acceptable excipient.

2. The oral liquid pharmaceutical solution according to claim 1, wherein one or more pharmaceutically acceptable excipient is selected from the group comprising co-solvents, solvents, antioxidants, microbial preservatives, buffering agents, aromatic agents, sweeteners or diluents.

3. The oral liquid pharmaceutical solution according to claim 2, wherein the microbial preservative is sodium benzoate.

4. The oral liquid pharmaceutical solution according to claim 2, wherein the diluent is maltitol solution.

5. The oral liquid pharmaceutical solution according to claim 2, wherein the sweetener is sucralose.

6. The oral liquid pharmaceutical solution according to claim 2, wherein the aromatic agent is orange aroma.

7. The oral liquid pharmaceutical solution according to any preceding claim, wherein gabapentin is in an amount of 1% to 10% of total weight of the pharmaceutical solution, more preferably 4% to 6% of total weight of the pharmaceutical solution.

8. The oral liquid pharmaceutical solution according to any preceding claim, wherein disodium EDTA is present in the range of 0.05% to 0.1% of total weight of the pharmaceutical solution, more preferably 0.09% to 0.1% of total weight of the pharmaceutical solution.

9. The oral liquid pharmaceutical solution according claims 3-8, sodium benzoate is present in the range of 0.01% to 0.1% of total weight of the pharmaceutical solution, more preferably 0.01% to 0.03% of total weight of the pharmaceutical solution.

10. The oral liquid pharmaceutical solution according to any preceding claim , the weight ratio of disodium EDTA to Gabapentin is 1:50

11. The oral liquid pharmaceutical solution according to claims 3-10 , the weight ratio of sodium benzoate to disodium EDTA is 1:5

12. The oral liquid pharmaceutical solution according to any preceding claim comprising;
a) 1 to 10% by weight of gabapentin,
b) 0.01 to 0.1% by weight of sodium benzoate,
c) 10 to 25% by weight of maltitol solution,
d) 0.05 to 0.1% by weight of disodium EDTA,
e) 0.05 to 0.5% by weight of sucralose,
f) 0.1 to 1 % by weight of orange aroma.

## Patentansprüche

1. Orale flüssige pharmazeutische Lösung, umfassend Gabapentin und Dinatrium-EDTA und mindestens einen pharmazeutisch verträglichen Exzipienten.

2. Orale flüssige pharmazeutische Lösung nach Anspruch 1, wobei ein oder mehrere pharmazeutisch verträgliche Exzipienten ausgewählt sind aus der Gruppe, umfassend Colösungsmittel, Lösungsmittel, Antioxidantien, mikrobielle Konservierungsmittel, Puffermittel, Aromastoffe, Süßungsmittel oder Verdünnungsmittel.

3. Orale flüssige pharmazeutische Lösung nach Anspruch 2, wobei das mikrobielle Konservierungsmittel Natriumbenzoat ist.

4. Orale flüssige pharmazeutische Lösung nach Anspruch 2, wobei das Verdünnungsmittel Maltitol-Lösung ist.

5. Orale flüssige pharmazeutische Lösung nach Anspruch 2, wobei das Süßungsmittel Sucralose ist.

6. Orale flüssige pharmazeutische Lösung nach Anspruch 2, wobei der Aromastoff Orangenaroma ist.

7. Orale flüssige pharmazeutische Lösung nach einem vorangehenden Anspruch, wobei Gabapentin in einer Menge von 1 % bis 10 % des Gesamtgewichts der pharmazeutischen Lösung, stärker bevorzugt 4 % bis 6 % des Gesamtgewichts der pharmazeutischen Lösung, vorliegt.

8. Orale flüssige pharmazeutische Lösung nach einem vorangehenden Anspruch, wobei Dinatrium-EDTA in dem Bereich von 0,05 % bis 0,1 % des Gesamtgewichts der pharmazeutischen Lösung, stärker bevorzugt 0,09 % bis 0,1 % des Gesamtgewichts der pharmazeutischen Lösung, vorliegt.

9. Orale flüssige pharmazeutische Lösung nach den Ansprüchen 3-8, wobei Natriumbenzoat in dem Bereich von 0,01 % bis 0,1 % des Gesamtgewichts der pharmazeutischen Lösung, stärker bevorzugt 0,01 % bis 0,03 % des Gesamtgewichts der pharmazeutischen Lösung, vorliegt.

10. Orale flüssige pharmazeutische Lösung nach einem vorangehenden Anspruch, wobei das Gewichtsverhältnis von Dinatrium-EDTA zu Gabapentin 1:50 beträgt.

11. Orale flüssige pharmazeutische Lösung nach den Ansprüchen 3-10, wobei das Gewichtsverhältnis von Natriumbenzoat zu Dinatrium-EDTA 1:5 beträgt.

12. Orale flüssige pharmazeutische Lösung nach einem vorangehenden Anspruch, umfassend:
a) 1 bis 10 Gew.-% Gabapentin,
b) 0,01 bis 0,1 Gew.-% Natriumbenzoat,
c) 10 bis 25 Gew.-% Maltitol-Lösung,
d) 0,05 bis 0,1 Gew.-% Dinatrium-EDTA,
e) 0,05 bis 0,5 Gew.-% Sucralose,
f) 0,1 bis 1 Gew.-% Orangenaroma.

## Revendications

1. Solution pharmaceutique liquide orale comprenant de la gabapentine et de l'EDTA disodique et au moins un excipient pharmaceutiquement acceptable.

2. Solution pharmaceutique liquide orale selon la revendication 1, dans laquelle un ou plusieurs excipients pharmaceutiquement acceptables sont choisis parmi le groupe comprenant les co-solvants, les solvants, les antioxydants, les conservateurs microbiens, les tampons, les agents aromatisants, les édulcorants ou les diluants.

3. Solution pharmaceutique liquide orale selon la revendication 2, dans laquelle le conservateur microbien est le benzoate de sodium.

4. Solution pharmaceutique liquide orale selon la revendication 2, dans laquelle le diluant est une solution de maltitol.

5. Solution pharmaceutique liquide orale selon la revendication 2, dans laquelle l'édulcorant est le sucralose.

6. Solution pharmaceutique liquide orale selon la revendication 2, dans laquelle l'agent aromatisant est un arôme d'orange.

7. Solution pharmaceutique liquide orale selon l'une quelconque des revendications précédentes, dans laquelle la gabapentine est comprise dans une quantité allant de 1 % à 10 % du poids total de la solution pharmaceutique, de préférence de 4 % à 6 % du poids total de la solution pharmaceutique.

8. Solution pharmaceutique liquide orale selon l'une quelconque des revendications précédentes, dans laquelle l'EDTA disodique est présent dans la plage allant de 0,05 % à 0,1 % du poids total de la solution pharmaceutique, de préférence de 0,09 % à 0,1 % du poids total de la solution pharmaceutique.

9. Solution pharmaceutique liquide orale selon l'une quelconque des revendications 3 à 8, dans laquelle le benzoate de sodium est présent dans la plage allant de 0,01 % à 0,1 % du poids total de la solution pharmaceutique, de préférence de 0,01 % à 0,03 % du poids total de la solution pharmaceutique.

10. Solution pharmaceutique liquide orale selon l'une quelconque des revendications précédentes, dans laquelle le rapport de poids EDTA disodique à gabapentine est de 1:50.

11. Solution pharmaceutique liquide orale selon l'une quelconque des revendications précédentes, dans laquelle le rapport de poids benzoate de sodium à EDTA disodique est de 1:5.

12. Solution pharmaceutique liquide orale selon l'une quelconque des revendications précédentes, comprenant :
a) 1 à 10 % en poids de gabapentine,
b) 0,01 à 0,1 % en poids de benzoate de sodium,
c) 10 à 25 % en poids de solution de maltitol,
d) 0,05 à 0,1 % en poids d'EDTA disodique,
e) 0,05 à 0,5 % en poids de sucralose,
f) 0,1 à 1 % en poids d'arôme d'orange.
